# EUROPEAN PATENT APPLICATION

(11) **EP 3 132 744 A1**
(43) Date of publication of application: **22.02.2017**
(21) Application number: 15780037.6
(22) Date of filing: 13.04.2015
(51) Int. Cl.: A61B 5/151

(54) **LANCET**

(30) Priority: 16.04.2014 JP 2014084356
(71) Applicant: Asahi Polyslider Company, Limited, Osaka 530-0005 (JP)
(72) Inventor: SAEKI, Hideaki, Maniwa-shi Okayama 719-3226 (JP); IMORI, Hirokazu, Maniwa-shi Okayama 719-3226 (JP)
(74) Representative: Hoffmann Eitle
(86) International application number: PCT/JP2015/061353
(87) International publication number: WO 2015/159845

(57) **Abstract**

There is provided a lancet wherein a lancet cap is composed at least of a grip member and a pricking-component protective member, and the pricking-component protective member has a two-part structure of a flexible part and a rigid part, the flexible part having a relative flexibility with respect to the rigid part, the rigid part having a relative rigidity with respect to the flexible part.

## Description

### TECHNICAL FIELD

The present invention relates to a lancet. More specifically, the present invention relates to a lancet which is used for taking a sample of body fluid (e.g., blood).

### BACKGROUND OF THE INVENTION

In order to measure a blood sugar level of patients with diabetes, it is required to take a sample of the blood from the patients. The small amount of the blood to be taken can be enough. Thus, a lancet for taking such small amount of the blood is used to measure the blood sugar level. The lancet is generally equipped with a pricking needle capable of puncturing a predetermined region of the individual's body (see, for example, U.S. Patent No. 5, 385, 571).

In general, the lancet is used in conjunction with an injector. The injector has a function of launching the lancet toward the predetermined region. By loading the lancet into the injector, a pricking device is set up for use. The lancet loaded into the injector is launched toward the predetermined region of the individual's body by means of a plunger of the injector, whereby the predetermined region is pricked.

### Prior Art Documents

### Patent Documents

Patent Document 1: U.S. Patent No. 5,385,571

### DISCLOSURE OF THE INVENTION

### PROBLEMS TO BE SOLVED BY THE INVENTION

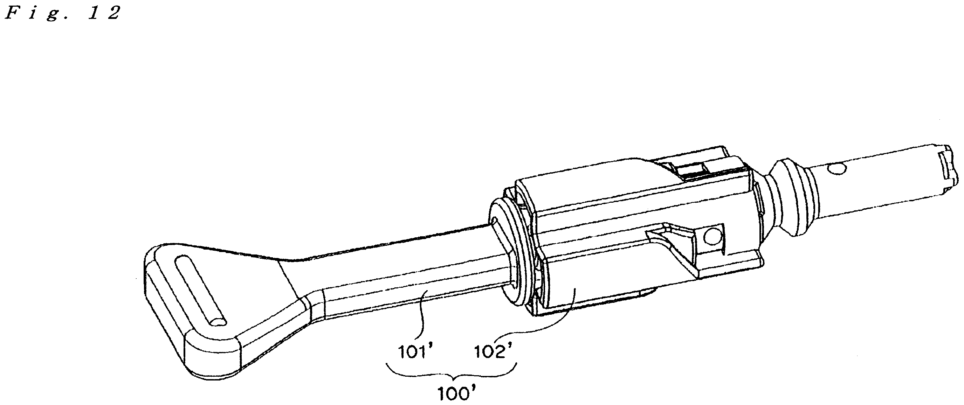

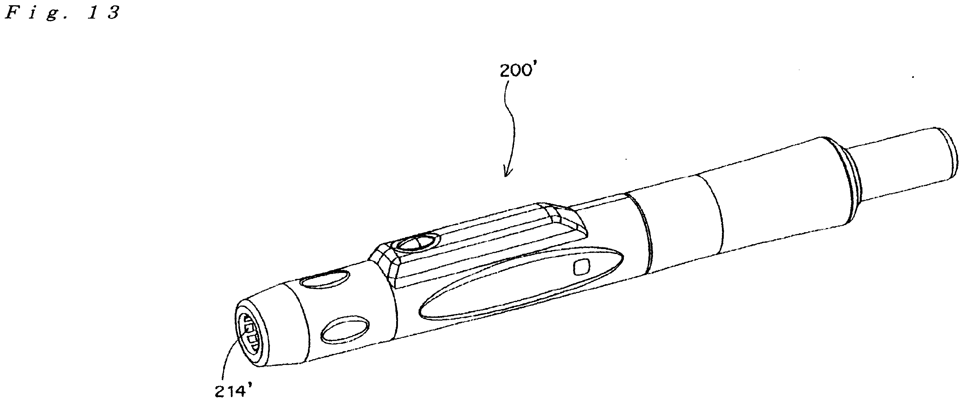

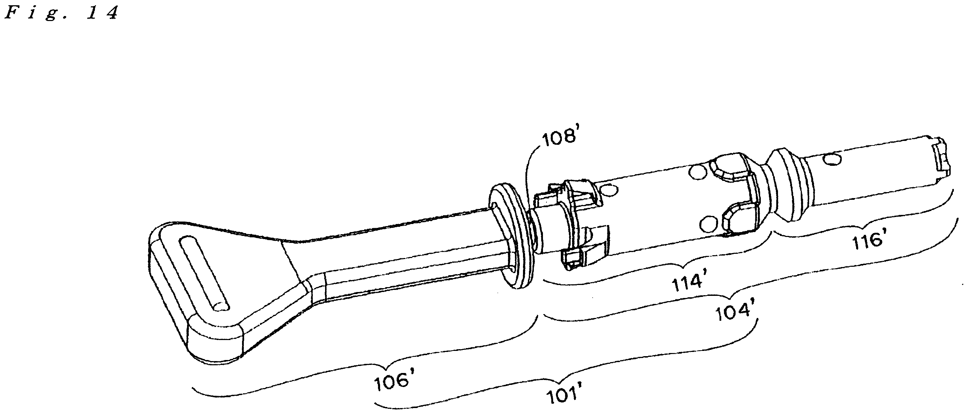

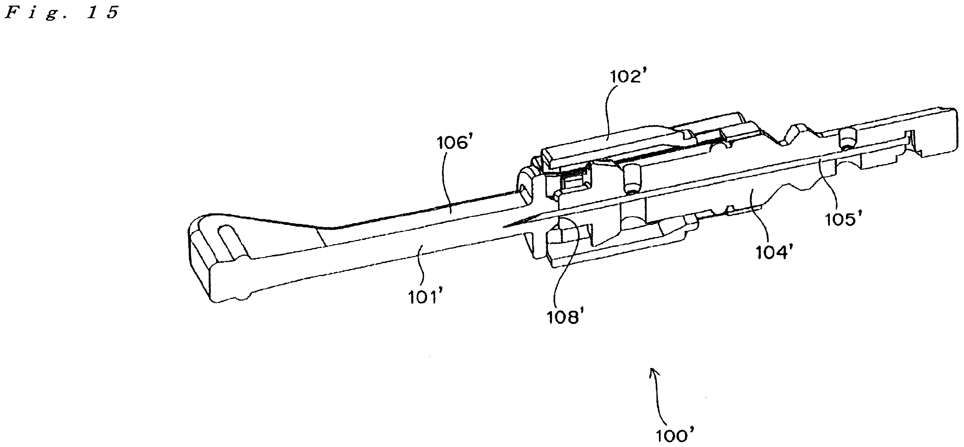

The present applicant has invented the following pricking device, and filed the application regarding such device (WO 2007/018215 A1, filed date: August 8 2006, title of the invention: "PRICKING DEVICE, AS WELL AS LANCET ASSEMBLY AND INJECTOR ASSEMBLY OF THE PRICKING DEVICE"). Referring to the accompanying drawings, the lancet assembly and the injector assembly invented by the applicant will be briefly explained below (note that the term "*lancet assembly"* will be hereinafter referred to also as "*lancet*", and the term *"injector assembly"* will be hereinafter referred to also as *"injector"*). Fig. 12 shows an external appearance of a lancet 100' , and Fig. 13 shows an external appearance of an injector 200' . As shown in Figs. 14 and 15, the lancet 100' is composed of a lancet body 104' , a lancet cap 106' and a pricking component 105' . The pricking component 105' is attached to the lancet body 104' , and the tip of the pricking component 105' is covered with the lancet cap 106'. The lancet cap 106' and the lancet body 104' are integrally connected together by a weakened part 108'. As shown in Figs. 12 and 15, the protective cover 102' is provided to enclose a part of the lancet body 104' . Such lancet 100' is loaded into the injector 200' for its use, followed by the lancet cap 106' being removed. By the removal of the lancet cap, the tip of the pricking component 105' is exposed so that the lancet becomes ready for pricking.

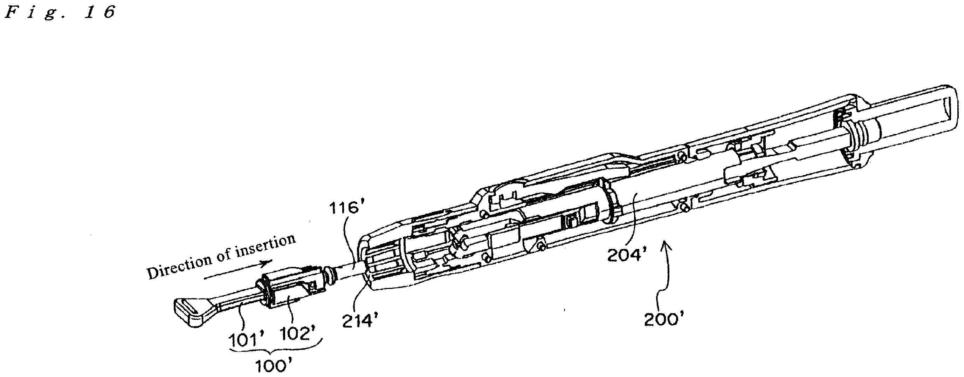

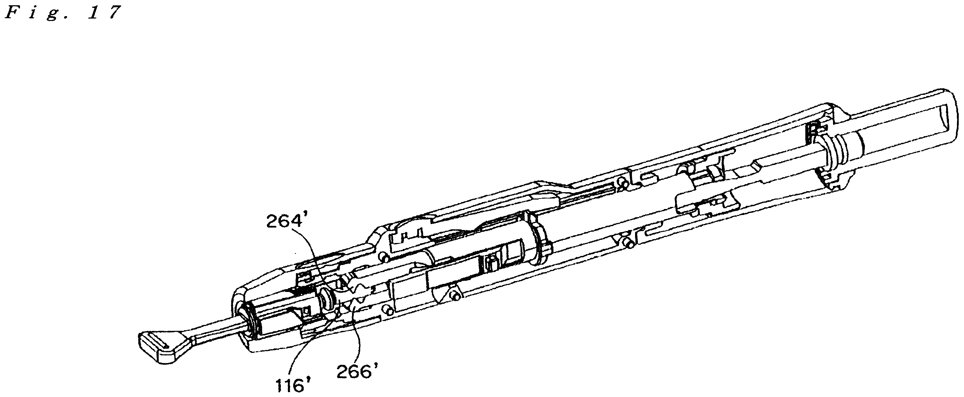

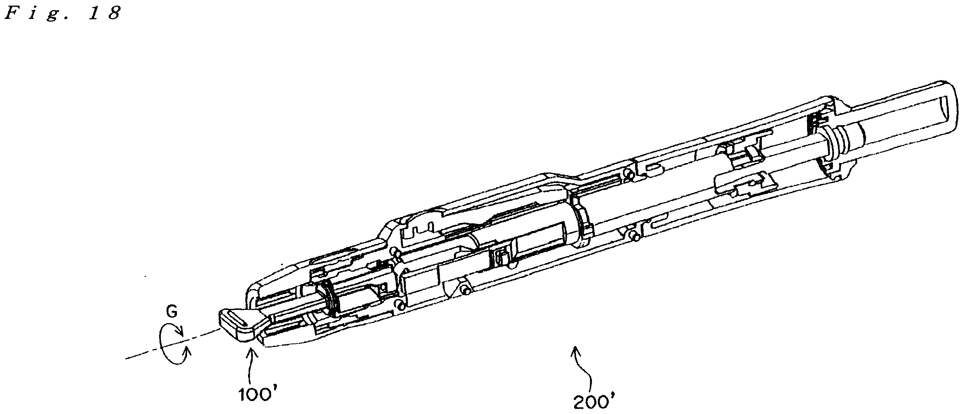

The injector 200' shown in Fig. 13 can be used in combination with the lancet 100' to launch the lancet body with the tip of the pricking component 105' exposed. The injector 200' comprises a plunger 204' . The plunger 204' is capable of engaging with a rear end portion of the lancet body to launch the lancet body in the pricking direction (see Fig. 16). As shown in Fig. 16, the lancet 100' is loaded into the injector 200' by inserting the lancet 100' into the injector 200' through a front end opening 214' of the injector 200' . When the inserting of the lancet proceeds to some degree, a rear portion 116' of the lancet 100' becomes held by tips 264' and 266' of the plunger 204' , as shown in Fig. 17. When the insertion of the lancet is further continued, the plunger 204' is thrusted backward so that the launching energy is stored. In other words, the retraction of the plunger 204' can cause a spring (not shown) provided in the plunger 204' to be compressed. This means that, when the compression of the spring is released, the plunger instantly moves forward to launch the lancet. Fig. 18 shows the injector 200' in the state where the plunger has retracted and the launching energy has been stored therein.

At a point in time after the completion of the loading of the lancet 100' into the injector 200' , the lancet cap 106' is removed to expose the tip of the pricking component 105' . The removal of the lancet cap 106' will be described as follows:
As shown in Figs. 14 and 15, the lancet body 104' and the lancet cap 106' are integrally connected together by the weakened part 108' located between the lancet body and the lancet cap. The weakened part 108' is forced to be broken upon a removal of the lancet cap 106', and thereby the lancet cap 106' can be separated from the lancet 100'.

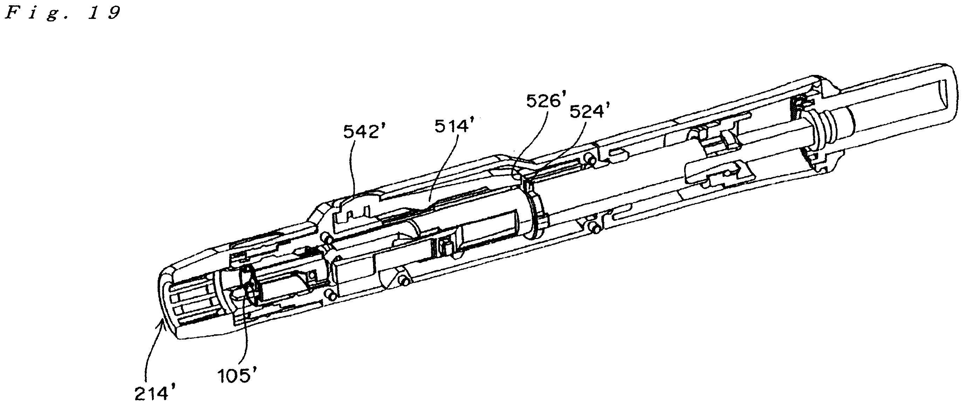

When the pricking operation is carried out, the front end opening 214' of the injector 200' is applied to a predetermined region to be pricked (for example, a finger tip of the individual). Subsequently, the press part 542' of a trigger component 514' is pushed. See Fig. 19. The pushing of the press part 542' causes an instantaneous expansion of the compressed spring, and thereby forcing the plunger 204' to move forwardly to prick the predetermined region with the pricking component.

Prior to the pricking operation, the lancet cap is removed from the lancet. To this end, a so-called "twist" operation is usually performed. Specifically, the lancet body 104' and the lancet cap 106' are rotated around the pricking component in the reverse direction to each other, causing the lancet body 104' and the lancet cap 106' to be separated from each other. Such removal of the lancet cap is preferable in that it can provide a high degree of freedom for user's operability since the rotation movement of the lancet cap as well as a pulling movement thereof in one direction is possible. However, the twist operation for the removal of the lancet cap can cause a great burden on the pricking component. The removal operation of the lancet cap can bring about a stress on the tip of the pricking component so that the tip of the pricking component warps. As a result, a concern can arise with respect to a distortion of the tip of the pricking component.

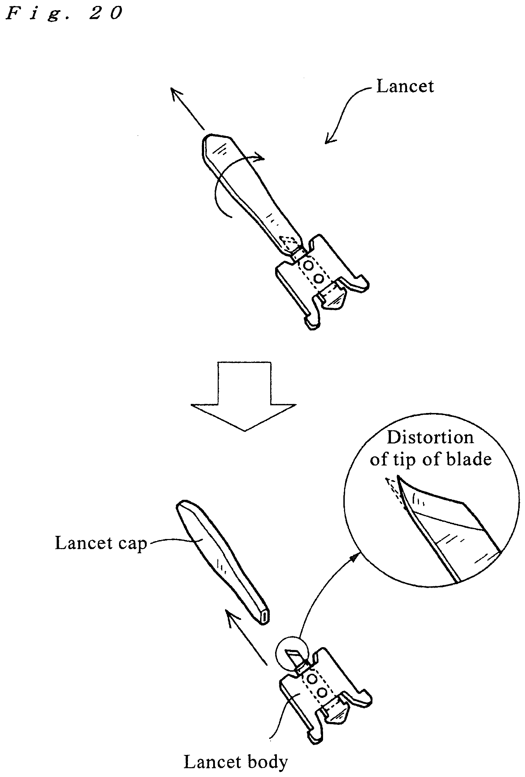

The inventors of the present application have found that, in a particular case where the pricking component has a form of blade, there can be generated a greater stress on the tip of the pricking component during the removal of the lancet cap, and thereby causing the distortion/curve of the tip of the pricking component to become too large to ignore. See Fig. 20.

The present invention has been created in view of the above-mentioned circumstances. That is, one of objects of the present invention is to provide a lancet capable of avoiding an inconvenience attributed to the removal of the lancet cap.

### MEANS FOR SOLVING THE PROBLEMS

In order to achieve the above object, the present invention provides a lancet comprising
a lancet body made of resin;
a lancet cap made of resin; and
a pricking component made of metal, the pricking component being arranged in the lancet body and the lancet cap such that a tip of the pricking component is covered by the lancet cap,
wherein the lancet cap is composed at least of a grip member and a pricking-component protective member, and
wherein the pricking-component protective member has a two-part structure of a flexible part and a rigid part, the flexible part having a relative flexibility with respect to the rigid part, the rigid part having a relative rigidity with respect to the flexible part.

One feature of the present invention consists in a structure of the lancet cap. Specifically, the lancet of the present invention is configured such that the lancet cap is composed at least of the grip member and the pricking-component protective member which has two-part structure of the flexible part and the rigid part. This structure of the pricking-component protective member with the flexible part being connected with the rigid part allows the lancet cap to locally warp, deform and/or break when a removing operation of the lancet cap is performed.

The term *"grip member"* as used herein substantially means a part of the lancet cap to be gripped by the user when a removal of the lancet cap is performed prior to the pricking operation.

The term *"pricking-component protective member"* as used herein substantially means a part of the lancet cap, the part enclosing the tip of the pricking component. As far as the relationship between *"grip member" and "pricking-component protective* member", a part other than the grip member in the lancet cap corresponds to the pricking-component protective member.

In the lancet according to a preferred embodiment of the invention, the "flexible part" is positioned inwardly with respect to the rigid part, and the "rigid part" is positioned outwardly with respect to the flexible part in the pricking-component protective part member. That is, the lancet cap as a whole has such a form that the flexible part is surrounded by the rigid part. More preferably, the rigid part surrounds the flexible part except for a body-adjacent side of the lancet cap, the side being adjacent to the lancet body.

In the lancet according to a preferred embodiment of the invention, the flexible part of the pricking-component protective member has such a form that a pair of plate-shaped members are opposed to each other. In particular, it is preferred that the tip of the pricking component is sandwiched by the opposed plate-shaped members. This means that the two plate-shaped members are opposed to each other with the interposition of the tip of the pricking component therebetween.

In the lancet according to a preferred embodiment of the invention, at least a part of a connecting portion of the flexible part and the rigid part has a groove form in the pricking-component protective member. This means that the connecting portion, which extends along the longitudinal direction of the lancet, preferably has the groove form.

In the lancet according to a preferred embodiment of the invention, the pricking component has a form of blade. That is, the pricking component in the form of blade is arranged in the lancet body and the lancet cap such that the tip of the blade is covered by the lancet cap.

In the lancet according to a preferred embodiment of the invention, the connecting portion of the flexible part and the rigid part, which has the form of groove, extends along a side periphery of the tip of the pricking component. This means that the connecting portion in the form of groove preferably extends to align with the side periphery (i.e., lateral side edge) of the tip of the pricking component such that the connecting portion and the side periphery are opposed to each other.

In the lancet according to a preferred embodiment of the invention, the lancet cap has the grip member and the pricking-component protective member which are integrally formed with each other. Furthermore, the flexible part and the rigid part are integrally formed with each other in the pricking-component protective member. That is, the pricking-component protective member of the lancet cap may have the two-part of the flexible part and the rigid part, the two-part being in a form of integration with each other.

In the lancet according to a preferred embodiment of the invention, the pricking-component protective member further has a beam portion on an upper surface of the flexible part, the beam portion being in a connection with the rigid part. That is, a beam-formed portion is provided in the flexible part.

In the lancet according to a preferred embodiment of the invention, the flexible part of the pricking-component protective member is capable of warping when such a removal of the lancet cap is performed that the lancet cap is away from the tip of the pricking component. That is, the removal of the lancet cap, which is performed prior to the pricking operation, can cause the flexible part to at least locally warp. It is preferred that the warping of the flexible part allows at least a part of a connecting portion of the flexible part and the rigid part to be eventually broken upon the removal of the lancet cap. It is preferred that a starting point of the break takes place at the connecting portion of the flexible part and the rigid part. In a particular case where the connecting portion of the flexible part and the rigid part has the form of groove, it is preferred that at least a part of the groove is broken. By way of example, the local break may occur in sub-connecting portions of the flexible part and the rigid part, the sub-connecting portions being diagonally opposed to each other.

### EFFECTS OF THE INVENTION

In accordance with the lancet of the invention wherein the pricking-component protective member of the lancet cap has the two-part structure of the relatively flexible part and the relatively rigid part, there can be suitably avoided an inconvenience attributed to the removal of the lancet cap. Specifically, when the lancet cap is removed, the flexible part of the pricking-component protective member is allowed to warp to reduce the stress applied on the tip of the pricking component, causing a prevention of the distortion/curve of the tip of the pricking component.

In a preferred embodiment, the removal of the lancet cap allows at least a part of a connecting portion of the flexible part and the rigid part to be eventually broken, which leads to a more reliable prevention of the distortion of the tip of the pricking component.

### BRIEF DESCRIPTION OF THE DRAWINGS

Fig. 1 is a perspective view of a lancet according to an embodiment of the present invention.
Fig. 2 includes plan and sectional views of a lancet according to an embodiment of the present invention.
Fig. 3 is a sectional perspective view of a lancet wherein half of the lancet has been cut away along the longitudinal direction thereof.
Fig. 4 includes a perspective view of a pricking-component protective member of a lancet cap.
Fig. 5 includes a perspective view of a pricking-component protective member of a lancet cap.
Fig. 6 includes a perspective view of a pricking-component protective member of a lancet cap wherein a connecting portion in a form of groove is provided.
Fig. 7 includes a perspective view of a pricking-component protective member of a lancet cap wherein a beam portion is additionally provided.
Fig. 8 includes a perspective view of a pricking-component protective member of a lancet cap wherein a flexible part is in a form of "thin skin".
Fig. 9 includes views of temporal change of a lancet at a point time during a removal of lancet cap is performed.
Fig. 10 includes views of temporal change of a lancet showing a breaking of a flexible part thereof.
Fig. 11 is an exploded perspective view of a lancet manufactured in [EXAMPLE] wherein a lancet body and a lancet cap are individually shown.
Fig. 12 is a perspective view showing an appearance of a lancet (Prior Art).
Fig. 13 is a perspective view showing an appearance of an injector (Prior Art).
Fig. 14 is a perspective view showing an appearance of a lancet (Prior Art).
Fig. 15 is a perspective view showing a lancet of Fig. 12, the lancet having been cut away in half so as to make it easy to understand the inside of the lancet (Prior Art).
Fig. 16 is a perspective view showing the state before a lancet is loaded into an injector (Prior Art).
Fig. 17 is a perspective view showing the state in which a lancet is held by the tip of a plunger upon loading a lancet (Prior Art).
Fig. 18 is a perspective view showing the state of completed loading of a lancet wherein a plunger cannot be retracted any more (Prior Art).
Fig. 19 is a perspective view showing the state in which a lancet cap has been removed and thus a lancet is ready for pricking (Prior Art).
Fig. 20 is a perspective view showing an embodiment of the lancet of the prior art wherein a distortion/curve of the tip of a pricking component occurs upon a removal of a lancet cap (Prior Art).

### MODES FOR CARRYING OUT THE INVENTION

The lancet of the present invention will be described in detail below with reference to the accompanying drawings.

The term "direction" as used throughout the claims and description is defined as follows: The direction in which side a lancet cap is positioned when viewed on the whole is regarded as a "forward" direction, whereas the direction in which side a lancet body is positioned when viewed on the whole is regarded as a "backward"/"rearward" direction. In other words, the "forward direction" corresponds to the direction in which a pricking component moves upon performing a pricking operation, and the reverse direction thereto is regarded as a "backward"/"rearward" direction. These directions are indicated in the drawings.

### 〈〈Lancet of Present Invention〉〉

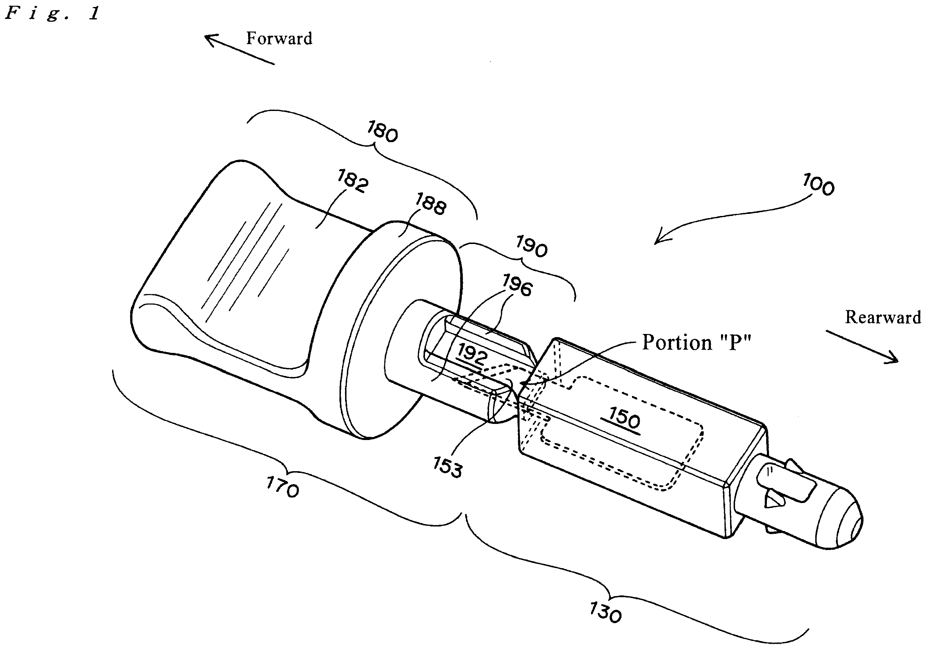

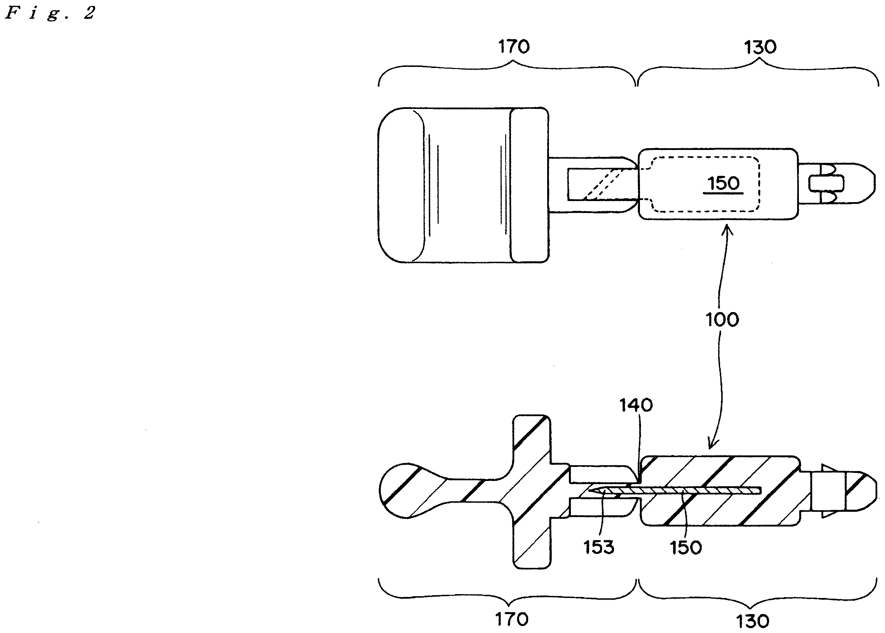

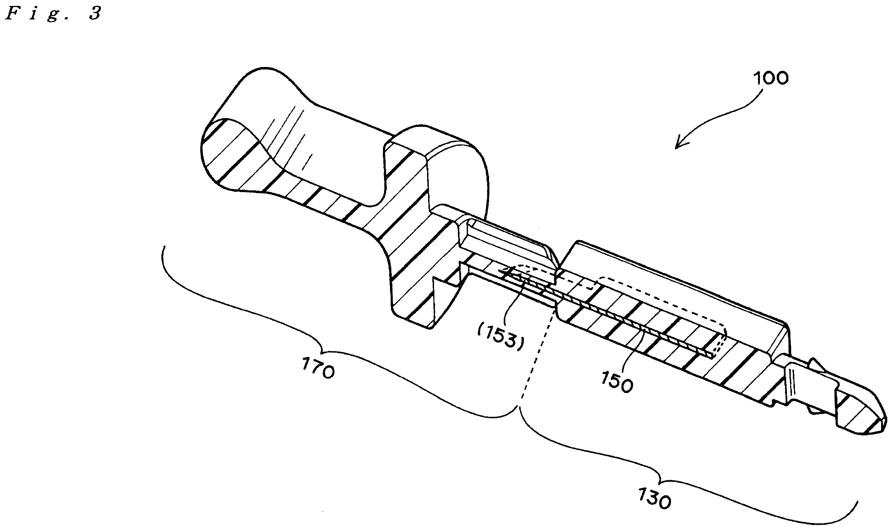

Fig. 1 shows an appearance of a lancet 100 according to an embodiment of the present invention. Fig. 2 shows plan and sectional views of the lancet 100 according to an embodiment of the present invention. Fig. 3 is a sectional perspective view of the lancet wherein half of the illustrated lancet has been cut away along the longitudinal direction thereof. As shown in Figs. 1-3, the lancet 100 of the present invention is mainly composed of a lancet body 130, a lancet cap 170 and a pricking component 150. The lancet body 130 and the lancet cap 170 are made of resin, whereas the pricking component 150 is made of metal.

It is preferred that the pricking component 150 has a form of "blade" at the tip thereof. The tip of the pricking component 150 with the form of blade is planarly sharp. This means that the tip of the pricking component 150 preferably has a flat shape as a whole (i.e., plate-like flatten shape), as shown in Fig. 1.

The pricking component 150 is situated within the both of the lancet body 130 and the lancet cap 170 such that the tip 153 of the pricking component 150 is covered with the lancet cap 170.

The lancet cap 170 and the lancet body 130 are integrally connected to each other via only a small contact portion. Such contact is indicated by reference numeral "140" in Fig. 2. The lancet 100 can be formed by a so-called insert molding process wherein the pricking component 150 is inserted into a metal mold. The contact portion can be formed upon carrying out such insert molding process. Accordingly, the contact portion can be made of the same resin as that of the lancet cap 170 and the lancet body 130. Suitable materials for the lancet body 130 and the lancet cap 170 are resin which is commonly used for the lancet in general, such as polyethylene and polypropylene. It is, however, preferred that the materials for the lancet body and the lancet cap are a soft resin. For example, such soft resin can be at least one kind of resin selected from the group consisting of a low density polyethylene, a high density polyethylene, a polystyrene and an elastomer. While on the other hand, the metals for the pricking component 150 may be any suitable ones which are also commonly used for a pricking needle of a lancet in general. The metal for the pricking component may be a stainless steel, for example.

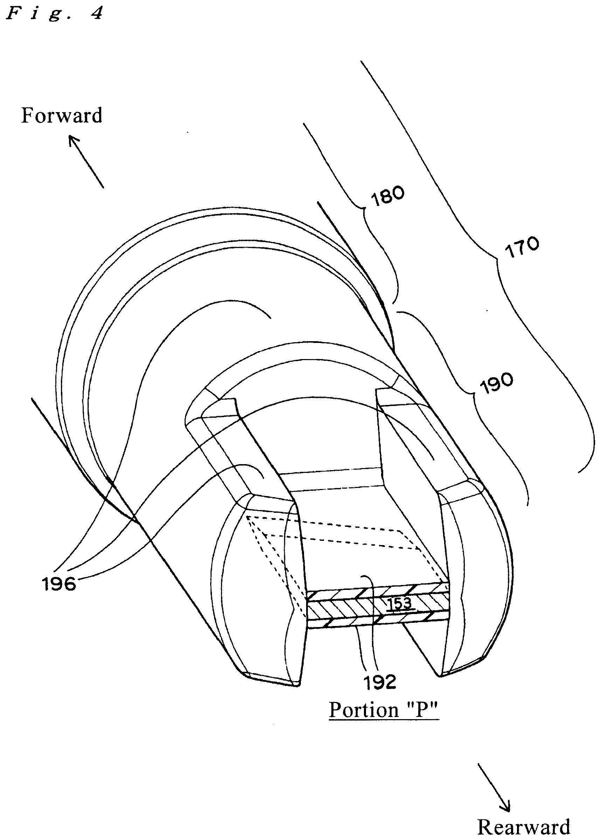

As shown in Figs. 1 and 4, the lancet 100 of the present invention comprises the lancet cap 170 composed at least of a grip member 180 and a pricking-component protective member 190, in which the pricking-component protective member 190 has a two-part structure of a flexible part 192 and a rigid part 196, the flexible part having a relative flexibility with respect to the rigid part, the rigid part having a relative rigidity with respect to the flexible part.

As can be seen from Figs. 1 and 4, the grip member 180 and the pricking-component protective member 190 are preferably in connection with each other in the longitudinal direction of the lancet. More specifically, the grip member 180 and the pricking-component protective member 190 are integrally connected with each other such that the grip member 180 is positioned forward with respect to the pricking-component protective member 190, whereas the pricking-component protective member 190 is positioned rearward with respect to the grip member 180. In other words, the grip member 180 and the pricking-component protective member 190 are in an integral and continuous form with each other such that they align along the longitudinal direction of the pricking component 150.

The grip member 180 corresponds to a part of the lancet cap to be gripped by the user when a removal of the lancet cap prior to the pricking operation is performed. As such, the grip member 180 has a suitable form for the user's gripping upon the removal of the lancet cap. For example, the grip member 180 at least has an approximately flatten portion 182 to which the user's finger can be suitably fitted, as shown in Fig. 1. As far as the illustrated grip member is concerned, there is provided a flange portion 188 at the rear of the approximately flatten portion 182 (see Fig. 1).

While on the other hand, the pricking-component protective member 190 corresponds to a part of the lancet cap, the part enclosing the tip 153 of the pricking component. As described above, the pricking-component protective member 190 is located at the rear side of the grip member 180 such that the pricking-component protective member 190 and the grip member 180 are in a continuous form with each other. In a case where the grip member 180 has the flange portion 188, the pricking-component protective member 190 is in a connection with the flange portion 188 (see Fig. 1). In other words, the lancet cap 170 has such a connection form that the approximately flatten portion 182 and the pricking-component protective member 190 are connected with each other via the flange portion 188.

Fig. 4 illustrates the two-part structure of the pricking-component protective member 190. The pricking-component protective member 190 comprises "flexible part 192 having a relative flexibility with respect to the rigid part" and "rigid part 196 having a relative rigidity with respect to the flexible part". As shown in Fig. 4, the pricking-component protective member 190 may have an approximately cylindrical shape as a whole, for example one wherein a part of the cylindrical shape is hollowed. In particular, the flexible part 192 of the pricking-component protective member 190 may have a hollowed form. This means that the pricking-component protective member 190 may have a local recess at the flexible part 192 thereof.

The term "*flexible part*" as used herein means a cap portion capable of warping due to an external force. That is, the flexible part corresponds to a portion of the cap to be deformed by the external force applied thereto. More specifically, a part of the pricking-component protective member, which is subject to a warping, deforming or breaking by a stress applied to the lancet cap upon the removal of the lancet cap from the lancet in the use of the lancet, can correspond to "flexible part". While on the other hand, the term *"rigid part"* as used herein means a cap portion which is substantially incapable of warping due to an external force. That is, the rigid part corresponds to a portion of the cap to be not substantially deformed by the external force applied thereto. More specifically, another part of the pricking-component protective member, which is not substantially subject to a warping, deforming or breaking by a stress applied to the lancet cap upon the removal of the lancet cap from the lancet in the use of the lancet, can correspond to "rigid part".

In the pricking-component protective part member 190, the flexible part 192 is positioned relatively inwardly, whereas the rigid part 196 is positioned relatively outwardly. In other words, the flexible part 192 is positioned inwardly with respect to the rigid part 196, whereas the rigid part 196 is positioned outwardly with respect to the flexible part 192. For example, the rigid part 196 is located to the outer side of the pricking component 153. This makes it possible for the flexible part 192 to more effectively warp/deform due to a stress applied to the lancet cap upon the removal of the cap. The warping/deforming of the flexible part 192 leads to a prevention of the distortion of the tip of the pricking component.

As shown in Fig. 4, the rigid part 196 surrounds the flexible part 192 in the pricking-component protective member 190. The flexible part 192 and the rigid part 196 are in an integral and continuous form with each other such that the flexible part 192 is surrounded by the rigid part 196. More specifically, it is preferred that the rigid part 196 surrounds the flexible part 192 except for a body-adjacent side of the lancet cap, the side being adjacent to the lancet body. The body-adjacent side, which is indicated by *Portion* "*P*" in Figs. 1 and 4, can correspond to the rear-most side among the four peripheral sides of the flexible part 192 viewed as a whole. In other words, it is preferred that the rigid part 196 is in connection with the periphery of the flexible part 192 except for the rear-sided portion of the periphery of the flexible part. This can also contribute to a prevention of the distortion of the tip of the pricking component. In other words, this also makes it possible for the flexible part 192 to more effectively warp/deform due to a stress applied to the lancet cap upon the removal of the cap. In this regard, the rear side of the flexible part 192 is allowed to more effectively warp/deform.

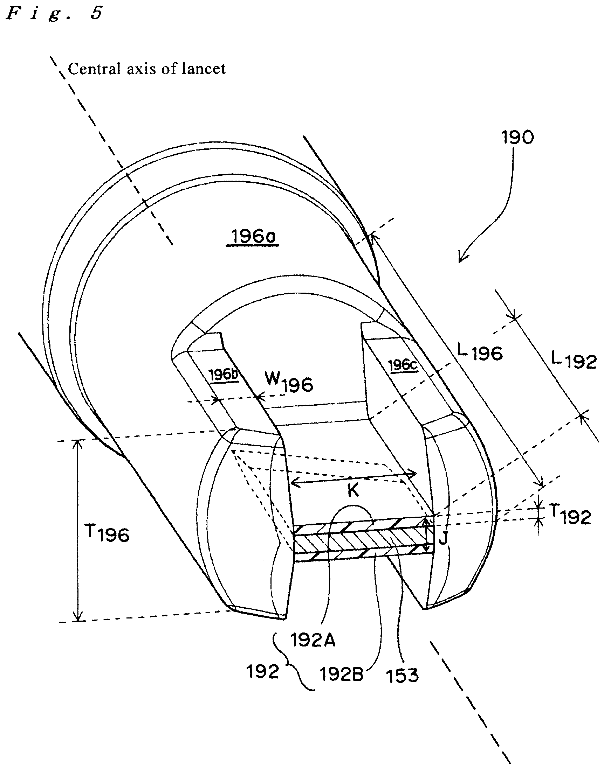

It is preferred that the flexible part 192 has such a form that a pair of plate-shaped members are opposed to each other. That is, as shown in Fig. 5, the plate-shaped sub-flexible parts 192A, 192B are preferably provided in such a parallel relationship that they are opposed to each other. More specifically, it is preferred that a pair of plate-shaped members 192A, 192B of the flexible part 192 are in an opposed arrangement to each other with the interposition of the tip 153 of the pricking component 150 therebetween. In particular, the principal surfaces of the tip 153 of the pricking component 150 are preferably sandwiched by the plate-shaped members 192A, 192B. In this regard, the plate-shaped member 192A is positioned on one of the principal surfaces of the tip 153, whereas the plate-shaped member 192B is positioned on the other of the principal surfaces of the tip 153. This configuration of the flexible part makes it possible for a stress to be more effectively applied to the lancet cap upon the removal of the cap, which will lead to more effective warping/deforming of the flexible part 192.

As shown in Fig. 5, it is preferred that the rigid part 196 is composed of a base portion 196a and a pair of sub-rigid parts 196b, 196c extending therefrom. The sub-rigid parts 196b, 196c extend rearward from the periphery of the base portion 196a. When the rigid part 196 is viewed from the lateral side thereof, the approximate shape of the rigid part 196 is like a Japanese katakana character "3" as a whole. As can be seen from Fig. 5, a pair of sub-rigid parts 196b, 196c are provided such that they interpose the flexible part 192 therebetween. More specifically, the plate-shaped members 192A, 192B are sandwiched by the sub-rigid parts 196b, 196c in the pricking-component protective member 190. In particular, the plate-shaped members 192A, 192B are sandwiched by the sub-rigid parts 196b, 196c such that lateral edges of the plate-shaped members 192A, 192B are in direct contact with the sub-rigid parts 196b, 196c. As far as the relationship between the rigid and flexible parts is concerned, the rigid part 196 and the flexible part 192 are preferably provided such that an opposing direction "J" of the plate-shaped members 192A, 192B is substantially perpendicular to an opposing direction "K" of the sub-rigid parts 196b, 196c (see Fig. 5).

As can be seen from the illustrated embodiments (for example the embodiment shown in Fig. 5), it is preferred that the pricking-component protective member 190 has a symmetrical form as a whole. In particular, it is preferred that the shape of the pricking-component protective member 190 is symmetric (bilaterally symmetric) to the central axis of the lancet.

The dimension of the flexible part, in particular each of the sub-flexible parts 192A, 192B (i.e., each of the plate-shaped members 192A, 192B) may have the approximate thickness "T₁₉₂" of 0.01 to 1.0 mm (see Fig. 5). The extending length "L₁₉₂" of each of the sub-flexible parts 192A, 192B (i.e., each of the plate-shaped members 192A, 192B) may be approximately in the range of 2 to 8 mm (see Fig. 5). In this regard, the dimension of the rigid part 196, in particular each of the sub-rigid parts 196b, 196c may have the approximate length "L₁₉₆" of 3.5 to 12 mm (see Fig. 5). The thickness "T₁₉₆" of each of the sub-rigid parts 196b, 196c may be approximately in the range of 1.5 to 6 mm (see Fig. 5). The width "W₁₉₆" of each of the sub-rigid parts 196b, 196c may be approximately in the range of 0.8 to 4 mm (see Fig. 5). It should be noted that the length, thickness and width respectively mean averaged values.

In the lancet according to a preferred embodiment of the invention, each thickness of the sub-flexible parts 192A, 192B (e.g., each thickness of the plate-shaped members 192A, 192B) is in the approximate range of 0.05 to 45 % of each thickness of the sub-rigid parts 196b, 196c, preferably in the approximate range of 0.1 to 30 % (for example 0.5 to 10 %) of each thickness of the sub-rigid parts 196b, 196c. On the whole wherein the thickness of the pricking component is presumed to be small similarly to that of the sub-flexible part, the flexible part 192 is thinner than the rigid part 196 such that the overall thickness of the flexible part is in the approximate range of 0.3 to 30 %, for example 1.5 to 10 % (particularly 10% or lower) of the thickness of the rigid part.

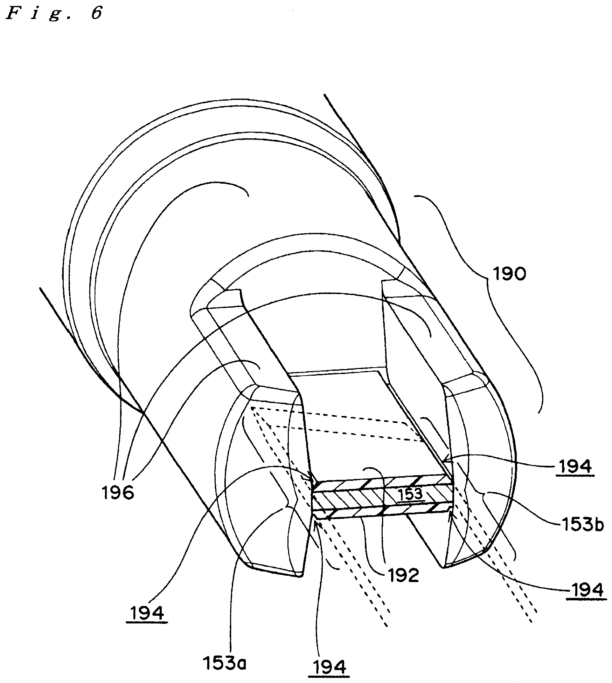

As shown in Fig. 6, it is preferred that at least a part of a connecting portion between the flexible part 192 and the rigid part 196 has a groove form in the pricking-component protective member 190. In particular, the part 194 of the connection portion, which is along the longitudinal direction of the lancet, preferably has the groove form. In other words, the flexible part 192 and the rigid part 196 are preferably in a connection with each other via the connection portion 194 (particularly via the part 194 of the connection portion being along the longitudinal direction of the lancet). This makes it possible for the connection portion 194 in the groove form to be broken by a stress applied to the lancet cap upon the removal of the cap, which will lead to a more effective warping/deforming of the flexible part 192. In this regard, the more effective warping/deforming of the flexible part 192 proceeds through the separation of the flexible part 192 and the rigid part 196 from each other at of the groove-formed connection portion 194, and thereby causing the flexible part 192 to more effectively warp/deform, which will lead to the effective prevention of the distortion of the tip of the pricking component. As for the embodiment of the connection portion having the groove form, at least a part of the connecting portion of the flexible part and the rigid part can be in a form of "*thin"*/"*thin skin".*

As shown in Fig. 6, the connection portion 194 in the groove form may be provided by a reduction in thickness of the edge of the flexible part 192 (specifically, the edges of sub-flexible parts 192A, 192B, more specifically the lateral edges thereof along the longitudinal direction of the lancet). This means that, as shown in Fig. 6, the local reduction of the thickness of the flexible part 192 at the contact portion with the rigid part (specifically, with the sub-rigid parts 196b, 196c) may bring about the groove form of the connection portion 194. This embodiment of the flexible part can also contribute to the more effective warping/deforming of the flexible part 192 at a point time during the removal of the lancet cap is performed, which will lead to a breaking of the flexible part at the groove-formed connection portion 194.

In order to more effectively promote the breaking of the connection portion 194 between the flexible part 192 and the rigid part 196, it is preferred that the connecting portion 194 in the form of groove extends along side peripheries 153a, 153b of the tip 153 of the pricking component (see Fig. 6). In this regard, the connecting portion 194 extending along the longitudinal direction (in particular each of four *"connecting portions 194"* extending along the longitudinal direction) preferably aligns with the side peripheries 153a, 153b of the tip of the pricking component (i.e., the edges 153a, 153b of the tip of the pricking component) such that the connecting portions are opposed to the side peripheries. In other words, the paired grooves or connecting portions 194 in the direction "K" have a spaced distance corresponding to a width dimension of the tip 153 of the pricking component.

In the pricking-component protective member 190 according to the present invention, it is preferred that the flexible part 192 and the rigid part 196 are integrally formed with each other. The lancet 100 can be formed by inserting the pricking component 150 into a metal mold in a so-called insert molding process, and thus the flexible part and the rigid part preferably become integrally formed with each other in the pricking-component protective member 190. In other words, the integration of the flexible part and the rigid part including the connecting portion thereof are provided in the lancet according to a preferred embodiment of the present invention. Furthermore, such integration of the flexible part and the rigid part including the connecting portion thereof are also integral with the lancet body. In a case where the flexible part and the rigid part are made of the same material as each other, the same material being typically attributed to the "integrally formed"/"integration", the flexibility and the rigidity in the pricking-component protective member 190 can be provided due to the form of the pricking-component protective member. For example, the difference in the thickness dimensions of the pricking-component protective member can bring about "flexibility" and "rigidity". More specifically, the flexibility and the rigidity can be provided due to the different thickness, in which case the flexible part has a relatively small thickness whereas the rigid part has a relatively large thickness.

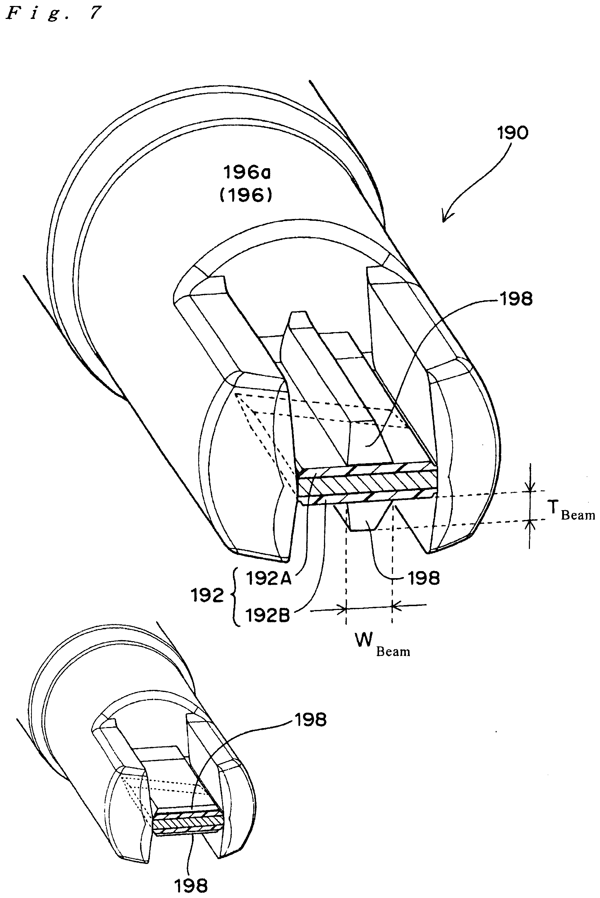

In addition to the embodiment of the lancet shown in Figs. 1-6 (particularly in addition to the embodiment of the pricking-component protective member thereof), another embodiment shown in Fig. 7 can also be conceivable. In the lancet of Fig. 7, the pricking-component protective member 190 further has a beam portion 198 on an upper surface of the flexible part 192. The beam portion 198 preferably extends in the longitudinal direction of the lancet such that the beam portion is in a connection with the rigid part 196 (the base portion 196a thereof in particular). The beam portion can contribute to a smooth removal of the lancet cap. Specifically, at a point in time when the tip of the pricking component is separated from the flexible part 192, the flexible part 192 is able to more reliably stay in the lancet cap while resisting the separation force applied to the flexible part. Namely, the beam portion 198 can effectively serve to prevent the flexible part 192 or a portion thereof from being stick to and thus remaining on the tip of the pricking component. The thickness T_{beam} of the beam portion 198 may be in the range of 0.5 to 10 times the thickness of the sub-flexible parts 192A, 192B, for example in the range of 1.5 to 8 times the thickness of the sub-flexible parts 192A, 192B (see Fig. 7). The width dimension W_{beam} of the beam portion 198 may be in the range of 0.5 to 2 times the thickness T_{beam} of the beam portion. In a case where the width dimension has a gradual change, the width dimension W_{beam} means an average dimension of width. In another preferred embodiment of the present invention, the beam portion 198 may have extremely large dimension of width (see the lower illustration of Fig. 7). In still another preferred embodiment of the present invention, the beam portion 198 may also have such a form that it extends along the central axis of the lancet (see Fig. 5).

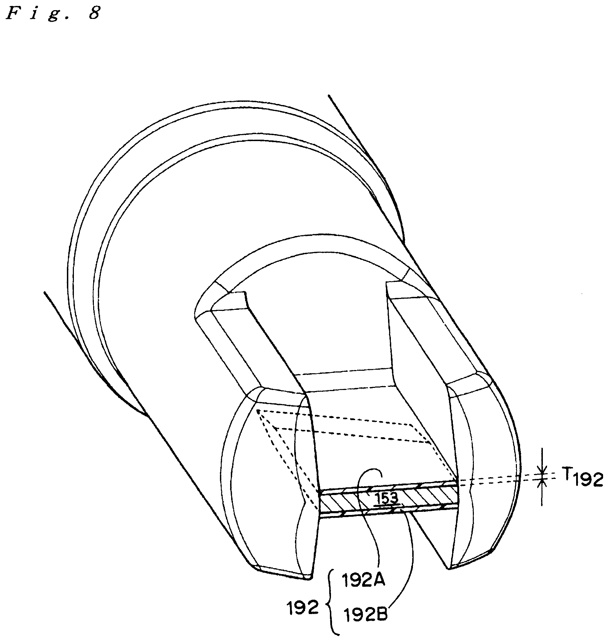

Still another embodiment shown in Fig. 8 can be conceivable with respect to the embodiment of the lancet (the pricking-component protective member thereof in particular). In the embodiment shown in Fig. 8, the flexible part may have extremely small dimension of thickness. For example, the thickness of the flexible part may be equal to or less than the thickness of the pricking component (e.g., the tip 153 thereof). In this regard, the flexible part may be provided on the tip face of the pricking component in a form of "*thin skin"* or "e*xtremely thin skin".* As such, the embodiment of Fig. 8 can correspond to an embodiment wherein the flexible part in a form of thin film is provided on the tip of the pricking component. More specifically, each thickness T₁₉₂ (Fig. 8) in a pair of the sub-flexible parts 192A, 192B (e.g., a pair of the plate-shaped members 192A, 192B) may be smaller by the approximate range of 10 to 60 %, for example 20 to 50 %, compared with those of Fig. 5.

### 〈〈Use Embodiment of Lancet〉〉

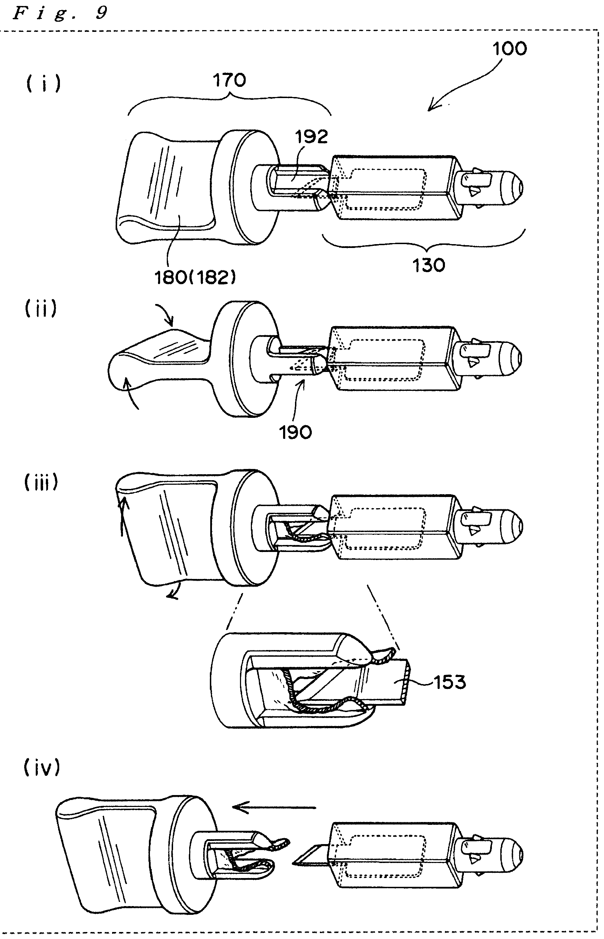

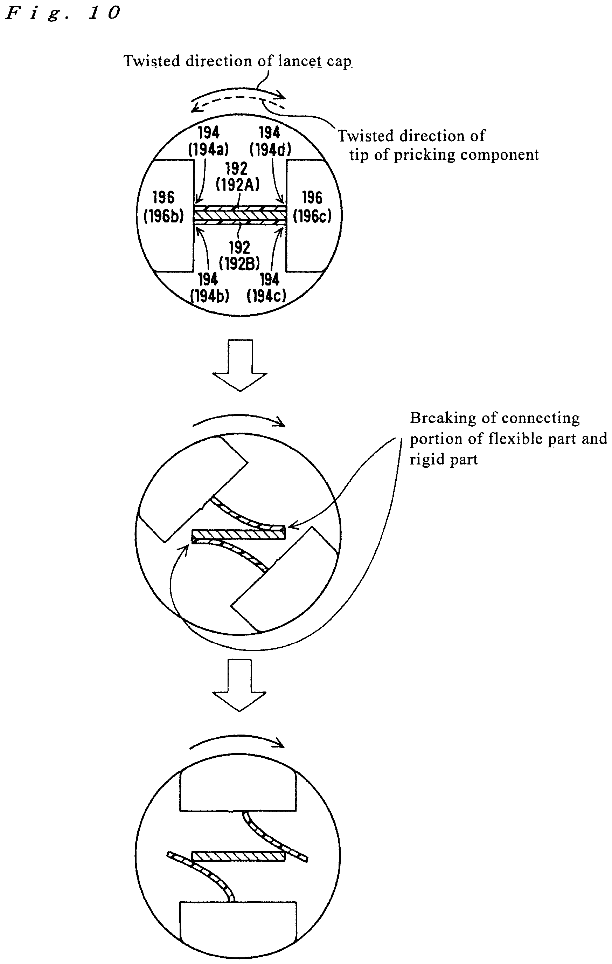

Now, the use embodiment of the lancet according to the present invention will be described. Figs. 9 and 10 respectively illustrate the use of lancet 100 over time. Fig. 9 illustrates perspective views of the lancet, and Fig. 10 illustrates sectional views of the lancet wherein the pricking-component protective member 190 has been cut away in the traverse direction of the lancet.

The lancet 100 of the present invention at a point time before the pricking operation is performed is shown in Fig. 9(i). For the pricking operation, the lancet cap 170 is removed from the lancet 100. The removal of the lancet cap 170 is preferably performed through a twisting of the lancet cap 170, as shown in Figs. 9(i)-9(iv). Specifically, the lancet cap 170 (the approximately flatten portion 182 of the grip member) is rotated around the longitudinal axis of the lancet so that a contact portion of the lancet body 130 and the lancet cap 170 is broken. Subsequent to the breaking of the contact portion, the lancet cap 170 is forced to move forward by being pulled with respect to the body. In other words, the grip member 182 of the lancet cap 170 is pulled while being twisted by the user's finger with the lancet body 130 being held.

According to one specific use embodiment of the lancet, the lancet 100 is loaded into an injector device (more specifically, the lancet body of the lancet is attached into a cylindrical lancet-attachment portion provided in the tip of a plunger of the injector device), and thereafter the pulling operation of the lancet cap is performed while the grip member 182 of the lancet cap 170 being twisted.

During the removal of the lancet cap, a stress is applied to the cap. The stress, however, can be effectively absorbed by the pricking-component protective member 190, which will lead to a reduction in the stress applied to the tip of the pricking component. Specifically, as shown in Figs. 9(ii) and 9(iii), when the grip member 180 is twisted, the flexible part 192 is allowed to more effectively warp/deform. In particular, the rear-sided portion of the flexible part 192 is allowed to more effectively warp/deform. As a result, there can be prevented the distortion of the tip of the pricking component upon the removal of the cap. The breaking of the flexible part 192 upon the removal of the cap is not necessarily required. The warping/deforming of the flexible part 192 with no breaking thereof can also reduce the stress applied to the tip of the pricking component, which will also lead to the prevention of the distortion/curve of the tip of the pricking component. In particular, the overall or local warping, or local deforming of the flexible part 192, which may occur due to the twisting of the cap, can also serve to reduce the stress applied to the tip of the pricking component. As a result, the distortion/curve of the tip of the pricking component can be prevented. As for the breaking of the flexible part 192, an adverse stress applied to the tip of the pricking component is unlikely to occur after the breaking of the flexible part 192. From this point of view, it is preferred that the flexible part 192 is capable of breaking upon the removal of the lancet cap (i.e., upon the twisting of the cap).

In a case where the connection portion of the flexible part 192 and the rigid part 196 has the groove form (in particular, the connection portion 194 extending along the longitudinal direction of the lancet has the groove form), at least a part of the grooved connection is capable of breaking upon the twisting of the grip member 182, which allows the flexible part 192 to more effectively warp/deform. More specifically, as shown in Fig. 10, the more effective warping/deforming of the flexible part 192 proceeds through the separation of the flexible part 192 and the rigid part 196 from each other at the grooved connection portion 194, which will lead to the more effective prevention of the distortion of the tip of the pricking component.

As shown in Fig. 10, it is preferred that the two grooved connection portions, which are among the four grooved connection portions and are diagonally opposed to each other, are partially broken upon the removal of the cap. As for the embodiment of Fig. 10, the diagonally opposed connection portions 194b and 194d, which are among the four connection portions 194a-194d in the groove form, are capable of partially breaking.

Although some embodiments of the present invention have been hereinbefore described, such embodiments are only for illustrative purpose as typical examples, and thus the present invention is not limited to these embodiments. It will be readily appreciated by those skilled in the art that various modifications are possible without departing from the scope of the invention. For example, the pricking component 150 has a "blade form" whose uppermost is wholly sharpened in the accompanying drawings, but is not necessarily limited thereto. The pricking component 150 may have a "needle form" i.e., a taper shape as long as the flexible part 192 is capable of effectively warping/deforming upon the removal of the lancet cap.

It should be noted that the present invention as described above includes the following aspects:
The first aspect: A lancet comprising
   a lancet body made of resin;
   a lancet cap made of resin; and
   a pricking component made of metal, the pricking component being arranged in the lancet body and the lancet cap such that a tip of the pricking component is covered by the lancet cap,
   wherein the lancet cap is composed at least of a grip member and a pricking-component protective member, and
   wherein the pricking-component protective member has a two-part structure of a flexible part and a rigid part, the flexible part having a relative flexibility with respect to the rigid part, the rigid part having a relative rigidity with respect to the flexible part.

The second aspect: The lancet according to the first aspect, wherein the flexible part is positioned inwardly with respect to the rigid part, and the rigid part is positioned outwardly with respect to the flexible part in the pricking-component protective part member.

The third aspect: The lancet according to the second aspect, wherein the rigid part surrounds the flexible part except for a body-adjacent side of the lancet cap, the side being adjacent to the lancet body.

The fourth aspect: The lancet according to any one of the first to third aspects, wherein the flexible part of the pricking-component protective member has a form of a pair of plate-shaped members which are opposed to each other, and
wherein the tip of the pricking component is sandwiched by the opposed plate-shaped members.

The fifth aspect: The lancet according to any one of the first to fourth aspects, wherein at least a part of a connecting portion of the flexible part and the rigid part has a groove form in the pricking-component protective member.

The sixth aspect: The lancet according to any one of the first to fifth aspects, wherein the pricking component has a form of blade.

The seventh aspect: The lancet according to the sixth aspect when appendant to the fifth aspect, wherein the connecting portion in the form of groove extends along a side periphery of the tip of the pricking component (i.e., periphery/edge of the tip of the pricking component, the periphery/edge extending in parallel with the longitudinal direction of the lancet).

The eighth aspect: The lancet according to any one of the first to seventh aspects, wherein the flexible part and the rigid part are integrally formed with each other.

The ninth aspect: The lancet according to any one of the first to eighth aspects, wherein the pricking-component protective member further has a beam portion on an upper surface of the flexible part, the beam portion being in a connection with the rigid part.

The tenth aspect: The lancet according to any one of the first to ninth aspects, wherein, upon a removal of the lancet cap from the tip of the pricking component, the flexible part warps to eventually break at least a part of a connecting portion of the flexible part and the rigid part.

### EXAMPLES

### 〈〈Actually Manufactured Lancet〉〉

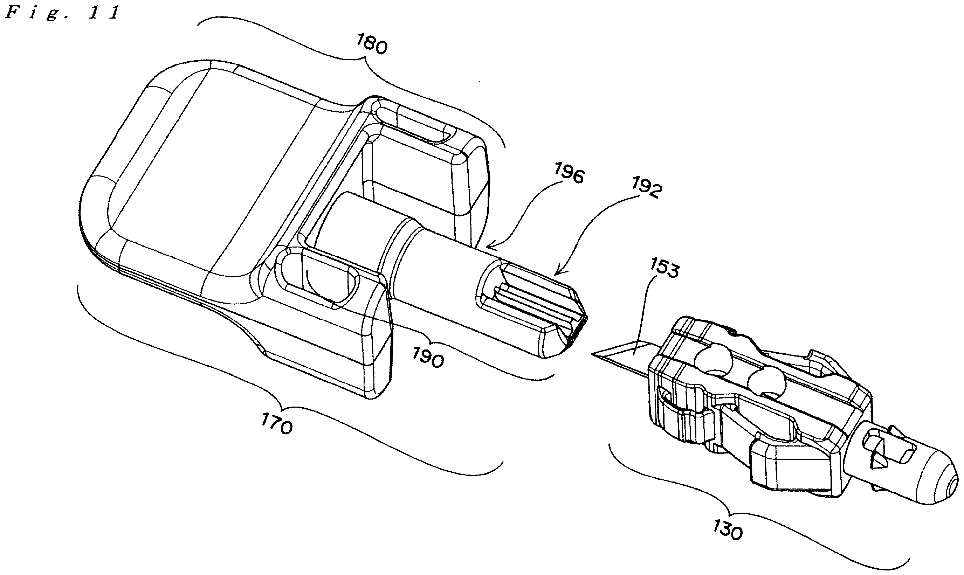

The lancet was manufactured according to the present invention (see Fig. 11). The lancet shown in Fig. 11 had the lancet cap composed at least of the grip member 180 and the pricking-component protective member 190 wherein the pricking-component protective member had a two-part structure of the flexible part 192 and the rigid part 196, the flexible part having a relative flexibility with respect to the rigid part, the rigid part having a relative rigidity with respect to the flexible part.

The removing operation of the lancet cap was performed with the grip member of the lancet cap being held by the user's finger. It was then confirmed that the removal of the cap allows the flexible part to warp, eventually causing a deformation or breaking of at least a part of the connection portion of the flexible part and the rigid part

At a point in time after the removal of the lancet cap, the tip of the pricking component was observed. As a result, there was found no "distortion"/"curve" of the tip of the pricking component.

### INDUSTRIAL APPLICABILITY

The lancet of the present invention can be used for taking the blood sample of diabetic, but the use of lancet is not limited to that. The lancet of the present invention can also be used in various applications which need blood samplings.

### CROSS REFERENCE TO RELATED PATENT APPLICATION

The present application claims the right of priority of Japanese Patent Application No.2014-84356 (filed on April 16, 2014, the title of the invention: "LANCET"), the disclosures of which are all incorporated herein by reference.

### REFERENCE NUMERALS

100 Lancet
130 Lancet body
140 Contact of lancet cap and lancet body
150 Pricking component
153 Tip of pricking component
153a, 153b Side periphery of tip of pricking component
170 Lancet cap
180 Grip member
182 Approximately flatten portion of grip member
188 Flange portion of grip member
190 Pricking-component protective member
192 Flexible part of pricking-component protective member
192A, 192B Pair of plate-shaped members (sub-flexible parts)
194 Connecting portion of flexible part and rigid part (for example, connecting portion having groove form)
194a-194d Groove-formed connecting portion
196 Rigid part of pricking-component protective member
196a-196c Sub-rigid parts
198 Beam portion
100' Lancet (Prior Art)
101' Lancet (Prior Art)
102' Protective cover (Prior Art)
104' Lancet body (Prior Art)
105' Pricking component (Prior Art)
106' Lancet cap (Prior Art)
108' Weakened part (Prior Art)
114' Front portion of Lancet body (Prior Art)
116' Rear portion of Lancet body (Prior Art)
200' Injector (Prior Art)
204' Plunger (Prior Art)
214' Front end opening of injector (Prior Art)
264' , 266' Tip of plunger (Prior Art)
514' Trigger component (Prior Art)
524' Projection of plunger (Prior Art)
526' Rear edge of trigger component (Prior Art)
542' Press part of trigger component (Prior Art)

## Claims

1. A lancet comprising
a lancet body made of resin;
a lancet cap made of resin; and
a pricking component made of metal, the pricking component being arranged in the lancet body and the lancet cap such that a tip of the pricking component is covered by the lancet cap,
wherein the lancet cap is composed at least of a grip member and a pricking-component protective member, and
wherein the pricking-component protective member has a two-part structure of a flexible part and a rigid part, the flexible part having a relative flexibility with respect to the rigid part, the rigid part having a relative rigidity with respect to the flexible part.

2. The lancet according to claim 1, wherein the flexible part is positioned inwardly with respect to the rigid part, and the rigid part is positioned outwardly with respect to the flexible part in the pricking-component protective part member.

3. The lancet according to claim 2, wherein the rigid part surrounds the flexible part except for a body-adjacent side of the lancet cap, the side being adjacent to the lancet body.

4. The lancet according to claim 1, wherein the flexible part of the pricking-component protective member has such a form that paired plate-shaped members are opposed to each other, and
wherein the tip of the pricking component is sandwiched by the opposed plate-shaped members.

5. The lancet according to claim 1, wherein at least a part of a connecting portion of the flexible part and the rigid part has a groove form in the pricking-component protective member.

6. The lancet according to claim 1, wherein the pricking component has a form of blade.

7. The lancet according to claim 6, wherein at least a part of a connecting portion of the flexible part and the rigid part has a form of groove in the pricking-component protective member, and
wherein the connecting portion in the form of groove extends along a side periphery of the tip of the pricking component.

8. The lancet according to claim 1, wherein the flexible part and the rigid part are integrally formed with each other.

9. The lancet according to claim 1, wherein the pricking-component protective member further has a beam portion on an upper surface of the flexible part, the beam portion being in a connection with the rigid part.

10. The lancet according to claim 1, wherein, upon a removal of the lancet cap from the tip of the pricking component, the flexible part is capable of warping such that at least a part of a connecting portion between the flexible part and the rigid part eventually breaks.
